# FASCICULE DE BREVET EUROPEEN

(11) **EP 0 585 146 B1**
(45) Date de publication et mention de la délivrance du brevet: **12.05.1999**
(21) Numéro de dépôt: 93401693.2
(22) Date de dépôt: 30.06.1993
(51) Int. Cl.: C08F 2/32, A61L 15/00

(54) **Perfectionnement pour la préparation de polyacrylates superabsorbants à faible teneur en monomères résiduels**
Verbesserung in der Herstellung von superabsorbierenden Polyacrylaten mit niedrigem Restmonomergehalt
Improvement in the preparation of super-absorbant polyacrylates with low residual monomer content

(30) Priorité: 12.08.1992 FR 9209961
(43) Date de publication de la demande: 02.03.1994
(73) Titulaire: ELF ATOCHEM S.A., 92800 Puteaux, Hauts-de-Seine (FR)
(72) Inventeur: Rebre, Shu Rong, F-94300 Vincennes (FR); Collette, Christian, F-75005 Paris (FR); Denie, Sandrine, F- 75017 Paris (FR)
(74) Mandataire: Haicour, Philippe

(56) Documents cités:
- EP-A- 0 441 507
- FR-A- 2 614 027
- CHEMICAL ABSTRACTS, vol. 111, no. 18, 30 Octobre 1989, Columbus, Ohio, US; abstract no. 155076, 'MANUFACTURE OF WATER-ABSORBENTS CONTAINING LOW RESIDUAL MONOMERS' page 50 ;colonne 2 ; & JP-A-01 103 644 (SANYO CHEM. IND. LTD.)

## Description

La présente invention est relative à la production de poudres de polymères acryliques susceptibles d'absorber d'importantes quantités d'eau ou de fluides aqueux.

On sait réaliser des matériaux doués d'une forte capacité d'absorption d'eau par polymérisation en solution ou en suspension inverse de monomères à insaturation éthylénique, plus particulièrement de monomères acryliques. Les poudres que l'on obtient ainsi gonflent fortement en présence d'eau, en donnant des gels à haute résistance mécanique. Ces propriétés sont mises à profit, entre autres, pour la fabrication d'articles sanitaires pour l'absorption et la rétention de liquides corporels.

Ces produits contiennent toutefois des quantités non négligeables de monomère. C'est un grave inconvénient, parce que ces monomères sont toxiques, ou pour le moins agressifs envers les muqueuses et la peau. Diverses solutions plus ou moins satisfaisantes ont été proposées pour diminuer la teneur en monomère résiduel des polymères acryliques hydrophiles: traitement par l'isopropanol (EP 0262380, Cassella), par l'hydroxylamine (US 4929717, Stockausen), par un acide aminé comme la lysine (US 4766173, Nalco), ou encore par des composés azoïques ou des peroxydes, (JP 1-103644, Sanyo). Sanyo enseigne notamment que l'on peut réduire la teneur en monomère au niveau de 500 ppm, soit en introduisant un couple oxydo-réducteur dans la masse en cours de polymérisation, soit en traitant le polymère formé, mais préalablement à sa dessiccation finale, de 0,01 à 100 ppm de peroxyde d'hydrogène par rapport au polymère (exprimé en poids sec), 0,01 ppm étant une limite inférieure en dessous de laquelle aucun effet sensible sur le monomère résiduel n'est plus observé, et 100 ppm étant une limite supérieure qui ne saurait être franchie sans altération du pouvoir absorbant du produit final.

Un perfectionnement important dans la production de telles poudres absorbantes est décrit dans le brevet européen EP 0441507. Il consiste à réaliser la polymérisation du monomère acrylique en au moins deux étapes. Dans une première étape, on réalise de façon classique une polymérisation en suspension inverse, polymérisation qui conduit à la formation d'un gel. Dans une seconde étape, on fait absorber à ce gel une nouvelle charge de monomère, et on en provoque la polymérisation au sein même du gel précédemment formé. On peut, le cas échéant, répéter cette séquence d'absorption/polymérisation. On obtient de cette façon des résines polymères appréciées pour leur granulométrie sensiblement plus forte que celle des résines obtenues par polymérisation en suspension inverse simple, pour leur vitesse de gonflement en présence d'eau, et pour le module élastique, la plasticité et la résistance au dégonflement sous pression sensiblement améliorés du gel qu'elles forment en présence d'eau.

Il est apparu qu'à condition que le procédé comporte une séquence finale de séparation du polymère comprenant une première élimination d'eau par distillation azéotropique et un séchage ultérieur, il était possible de réduire la teneur en monomère résiduel du produit fini à un niveau aussi bas que 50 ppm en introduisant de l'eau oxygénée entre la distillation azéotropique et le séchage. Un résultat comparable n'est pas atteint si l'on agit à un autre endroit du procédé selon la revendication 1.

Dans les procédés d'obtention des superabsorbants acryliques par polymérisation en deux étapes, on utilise généralement des tensioactifs non ioniques tels que les esters d'acides gras et de sorbitan, de polyglycérine, de sucrose, ou les polyoxyéthylène alkylphényl éthers. Mais on a remarqué qu'il était industriellement très avantageux de pouvoir opérer la phase d'absorption du procédé multi-étapes sans qu'il soit nécessaire de refroidir très fortement le réacteur. Réaliser cette absorption à des températures de 35 à 45°C améliore très sensiblement la rentabilité du procédé, mais pour cela il est nécessaire d'avoir recours, pour la phase de première polymérisation en suspension inverse, à des tensioactifs spéciaux. Ce sont soit des tensioactifs de type copolymères-blocs selon la revendication 4, par exemple de polyéthylèneglycol/dodécylglycol formés d'une chaîne polyéthylèneglycol munie à l'une et/ou l'autre de leurs extrémités de plusieurs chaînes hydrophobes constituées de restes dodécylglycol et notamment des copolymères blocs polyéthylèneglycol/dodécylglycol, grâce auxquels on peut procéder à l'absorption de la seconde charge de monomère par le gel polymère formé en premier lieu à des températures de l'ordre de 35°C, soit des tensioactifs de type polymérisable répondant à la formule générale:

R₁-O-(CH₂-CH₂-O)ₙ-R₂

où R₁ est une chaîne hydrocarbonée à au moins 9 atomes de carbone,
où R₂ est un groupement polymérisable, reste acryloyle, méthacryloyle ou maléoyle,
et où le degré de condensation n en oxyde d'éthylène est compris entre 30 et 70, tensioactifs grâce auxquels la phase d'absorption de la seconde charge de monomère peut être conduite à une température de 35 à 45°C. Le monoester maléique du nonylphénol oxyéthyléné à 50 molécules d'oxyde d'éthylène est un tensioactif préféré de ce type. Il est introduit dans la phase aqueuse.

On a également montré que dans ces procédés multi-étapes, il était intéressant de jouer sur la neutralisation des phases aqueuses de monomère acrylique et d'utiliser comme phase à polymériser en premier lieu en suspension inverse un acide acrylique monomère surneutralisé par rapport au taux de neutralisation visé pour le polymère final, et comme phase à polymériser après absorption par le gel ainsi formé, un acide acrylique monomère sous-neutralisé, par exemple, selon le mode de réalisation de l'invention de la revendication 3. Le produit qui en résulte est une poudre constituée, non plus de sphères lisses, mais de particules sphéroïdales à surface irrégulière dont la forme n'est pas sans rappeler celle des truffes, de forte granulométrie comprise entre 100 et 500 µm, avec des taux de passant à 100 µm très réduits, en tous cas inférieurs à 0,5 % en poids, ce qui est très appréciable au niveau de la fabrication puisqu'on produit directement les grosses particules qui sont les seules utilisables pour les applications courantes et qu'on économise une séquence de post-agglomération. Au niveau de l'utilisation, la géométrie irrégulière des particules en assure une meilleure fixation dans le milieu fibreux et une plus grande facilité de mise en oeuvre. Ces produits ne sont pas pour autant dépourvu de monomères résiduels et il est très intéressant de leur appliquer le procédé de l'invention pour en ramener la teneur à 50ppm ou moins. Les poudres qui en résultent, et qui sont constituées de particules sphéroïdales à surface irrégulière d'acide polyacrylique neutralisé à un taux compris entre 65 et 95 %, de diamètre moyen compris entre 100 et 500 µm, avec un passant à 100 µm inférieur à 0,5 %, et dont la teneur en monomères résiduels est tout au plus égale à 50 ppm, font partie de l'invention.

Les quantités utiles de peroxyde d'hydrogène sont comprises entre 0,08 et 0,19 % par rapport au polymère sec. On l'introduit après dilution dans le réacteur après avoir opéré la déshydratation azéotropique et avant le séchage définitif du polymère. Les exemples suivants feront mieux comprendre l'invention.

### EXEMPLES

Dans les exemples qui suivent, on distingue les séquences :
a) de préparation de la phase solvant,
b) de préparation de la phase aqueuse de monomère (charge I)
c) de mise en suspension du monomère et polymérisation I,
d) de préparation de la phase aqueuse de monomère (charge Il),
e) d'absorption de la charge Il et de polymérisation II,
f) de séparation du polymère.
les termes "Hi-wax 1105A", "Tokusil P", "Aerosol MEM-NP50" et "Daprol E348" sont des marques commerciales.

### Exemple 1 (art antérieur)

L'exemple correspond à l'obtention d'acide polyacrylique neutralisé à 75 % selon le procédé ordinaire de polymérisation en suspension inverse ; il n'y a donc ici ni séquences d ni séquence e.
- Séquence a: dans un réacteur d'un litre muni d'un dispositif d'introduction de réactifs solides ou liquides, d'un agitateur à hélice ou à ancre, d'une sonde de température, d'un système de balayage par gaz neutre et d'un dispositif de chauffage/réfrigération, on dissout vers 80°C et sous agitation de 325 tours/minute, 1,38 g de polyéthylène modifié par l'anhydride maléique, un produit commercialisé par Mitsui Petrochemical Industries Co sous le nom de Hi-Wax 1105A et 0,92 g de di/tristéarate de saccharose dans 376 g d'heptane.
- Séquence b: à part on neutralise, en maintenant la température inférieure à 30°C, 92 g d'une solution aqueuse à 80 % en poids d'acide acrylique par 141,6 g de lessive de soude à 21,66 %. On ajoute 0,276 d'hydroxyéthylcellulose, puis Il g d'une solution aqueuse à 0,95 % de persulfate de potassium.
- Séquence c: le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation et maintenue à ce niveau pendant 30 minutes environ. La température est alors ramenée à 20°C.
- Séquence finale f: on provoque tout d'abord l'agglomération des particules de polymère en ajoutant 0.55 g de silice colloïdale (Tokusil P) délayée dans 45 g d'heptane. On procède à 60°-70°C avec agitation sous 1500 tours/minute. On élimine ensuite l'heptane et la majeure partie de l'eau par distillation. On ajoute alors au contenu du réacteur 9,2 g d'une solution aqueuse à 2% d'éthylène glycol diglycidyléther et on poursuit le séchage.

La poudre que l'on obtient ainsi a un passant à 100 µm de 2%, et une teneur en monomère acrylique inacceptable de 100 ppm, due aux quantités modestes de persulfate de potassium utilisées dans la séquence b.

### Exemple 2 (art antérieur)

L'exemple correspond à l'obtention de polyacrylate neutralisé à 75 % selon le procédé en deux étapes première polymérisation en suspension inverse/absorption seconde polymérisation dans le gel.
- Séquence a : dans le dispositif décrit à l'exemple 1, on dissout à 80°C et sous agitation de 400 tours/minute, 0,92 g de polyéthylène modifié Hi-Wax 1105A et 0,736 g de di/tristéarate de saccharose dans 376 g d'heptane.
- Séquence b: à part, on neutralise 92 g d'une solution aqueuse à 80 % en poids d'acide acrylique par 176,8 g de lessive de soude à 17,35 %. On ajoute 0,276 g d'hydroxyéthylcellulose, puis 5,5 d'une solution aqueuse à 1 % de persulfate de potassium, 0,92 d'une solution aqueuse à 2 % d'éthylène glycol diglycidyléther. Cette phase aqueuse est ramenée à 38°C.
- Séquence c: le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation et maintenue à ce niveau pendant environ 30 minutes. La température est alors ramenée à 20°C.
- Séquence d: pendant le déroulement de l'opération précédente, on procède à part à la neutralisation de 92 g de solution aqueuse à 80 % en poids d'acide acrylique par 176,8 g de lessive de soude à 17,35 %, puis on ajoute 5,5 d'une solution aqueuse à 1 % de persulfate de potassium et 0,92 g d'une solution aqueuse à 2 % d'éthylène glycol diglycidyléther. Cette phase aqueuse qui constitue la charge Il de monomère est alors ramenée à 20°C.
- Séquence e: l'agitation dans le réacteur est portée à 80 tours/minutes, le balayage d'azote étant maintenu à 80 litres/minutes. On y introduit peu à peu la charge Il, après quoi la température est portée à 70°C pour engager la seconde phase de polymérisation. On laisse la polymérisation se poursuivre pendant 30 minutes environ.
- Séquence finale f : on ajoute au contenu du réacteur 9,2 g d'une solution aqueuse à 2 % d'éthylène glycol diglycidyléther et on termine le séchage du produit.

La poudre que l'on obtient contient 1 % de passant à 100 µm et du monomère libre à la teneur élevée de 414 ppm.

### Exemple 3 (Comparatif)

Contre-exemple, qui ne diffère de l'exemple 2 que par introduction, au cours de la séquence d de préparation de la deuxième charge aqueuse de monomère acrylique, de 5,5 d'eau oxygénée à 1 % en même temps que le persulfate de potassium et l'éthylène glycol diglycidyléther.

La poudre ainsi obtenue a un passant à 100 pm de 1 % et sa teneur en monomère acrylique libre est restée très élevée (490 ppm).

### Exemple 4

Cet exemple selon l'invention ne diffère de l'exemple 2 que par introduction, entre la phase de déshydratation et la phase de séchage de la séquence f, de 0,552 g d'eau oxygénée à 20 % en même temps que les 9,2 g d'éthylène glycol diglycidyléther à 2 %.

On obtient ainsi une poudre dont le passant à 100µm est d'environ 1 % et dont la teneur en monomère acrylique libre est maintenant tombée à 30ppm.

### Exemple 5

On a combiné ici les avantages d'un procédé d'obtention de superabsorbant polyacrylique par polymérisation en suspension inverse/absorption-polymérisation dans le gel dans lequel on a réalisé l'absorption de la seconde charge de monomère à 35°C, avec l'introduction d'eau oxygénée entre la phase de déshydratation du polymère et son séchage.
- Séquence a: dans le dispositif décrit à l'exemple 1, on dissout à 80°C et sous agitation de 400 tours/minute, 0,92 g de polyéthylène modifié Hi-Wax 1105A dans 265,6 g d'heptane.
- Séquence b: à part, on neutralise 92 g d'une solution aqueuse à 80 % en poids d'acide acrylique par 139,4 g de lessive de soude à 22 %. On ajoute 0,276 g d'hydroxyéthylcellulose, puis 5,5 d'une solution aqueuse à 1 % de persulfate de potassium, 0,92 g d'une solution aqueuse à 2 % d'éthylène glycol diglycidyléther et 0,18 g de maléate ester de nonylphénol à 50 moles d'oxyde d'éthylène (Aerosol MEM-NP 50 de Cyanamid). A noter que cet émulsifiant est introduit dans le système en solution dans la charge aqueuse, ce qui est assez inhabituel quand on veut provoquer la formation d'une suspension inverse.
- Séquence c: le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation et maintenue à ce niveau pendant environ 30 minutes. La température est alors ramenée à 35°C.
- Séquence d: pendant le déroulement de l'opération précédente, on procède à part à la neutralisation de 92 de solution aqueuse à 80 % en poids d'acide acrylique par 139,4 g de lessive de soude à 22 %, puis on ajoute 5,5 g d'une solution aqueuse à 1 % de persulfate de potassium et 0,92 g d'une solution aqueuse à 2 % d'éthylène glycol diglycidyléther. Cette phase aqueuse qui constitue la charge Il de monomère est alors ramenée à 15°C.
- Séquence e: l'agitation dans le réacteur est portée à 800 tours/minutes, le balayage d'azote étant maintenu à 80 litres/minutes. On y introduit peu à peu la charge Il, après quoi la température est portée à 70°C pour engager la seconde phase de polymérisation. On laisse la polymérisation se poursuivre pendant environ 30 minutes.
- Séquence finale f: on ajoute au contenu du réacteur une solution formée de 1,84 g d'une solution aqueuse à 2 % d'éthylène glycol diglycidyléther et de 0,552 g d'eau oxygénée à 20 %, après quoi on poursuit le séchage du produit.

Le passant à 100 µm de la poudre ainsi obtenue est de 1 %. Sa teneur en monomère résiduel est de 41 ppm.

### Exemple 6

Dans cet exemple, on cumule l'avantage de la combinaison d'une surneutralisation de la première charge de monomère et d'une sous-neutralisation de la seconde charge, de l'utilisation d'un tensioactif de type polymère bloc qui autorise de procéder à l'absorption de la seconde charge de monomère par le gel formé au cours de la première polymérisation à une température relativement élevée de 35°C, et de la réduction du taux de monomère résiduel selon l'invention.
- Séquence a: en utilisant le dispositif de l'exemple 1, on dissout à 80°C et sous agitation de 400 tours/minute, 0,92 g de polyéthylène modifié Hi-Wax 1105A et 0,46 g d'un copolymère bloc polyéthylèneglycol/dodécylglycol, le Dapral E348, de AKZO (molécule à environ trois restes dodécylglycol à une extrémité d'une chaîne méthoxypolyéthylène glycol à environ 22 maillons éthylène-glycol), dans 285,1 g d'heptane.
- Séquence b : à part, on neutralise 92 g d'une solution aqueuse à 80 % en poids d'acide acrylique par 160,65 g de lessive de soude à 24,17 %. On ajoute 0,276 g d'hydroxyéthylcellulose, puis 2,75 g d'une solution aqueuse à 2 % de persulfate de potassium, 0,92 g d'une solution aqueuse à 2 % d'éthylène glycol diglycidyléther.
- Séquence c : le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation et maintenue à ce niveau pendant 30 minutes environ. La température est alors ramenée à 42°C.
- Séquence d : pendant le déroulement de l'opération précédente, on procède à part à la neutralisation de 92 g de solution aqueuse à 80 % en poids d'acide acrylique par 135,75 g de lessive de soude à 16,6 %, puis 2,75 g d'une solution aqueuse à 2 % de persulfate de potassium et 0,92 g d'une solution aqueuse à 2 % d'éthylène glycol diglycidyléther. Cette phase aqueuse qui constitue la charge II de monomère est alors ramenée à 15°C. L'agitation dans le réacteur est portée à 80 tours/minutes, le balayage d'azote étant maintenu à 80 litres/minutes. On y introduit peu à peu la charge II, après quoi la température est portée à 70°C pour engager la seconde phase de polymérisation. On laisse la polymérisation se poursuivre pendant 30 minutes environ.
- Séquence f : après la déshydratation azéotropique, on introduit dans le réacteur 0,92 g de solution aqueuse à 2 % d'éthylène glycol diglycidyléther et 0,552 g d'eau oxygénée à 20 %.

La poudre que l'on obtient ainsi est constituée de particules sphéroïdales à surface irrégulière, avec un passant à 100 µm pratiquement nul; sa teneur en monomère libre est de 45 ppm.

## Revendications

1. Procédé pour la réalisation de poudres superabsorbantes pour l'eau et les liquides aqueux constitués d'acide polyacrylique partiellement neutralisé, à teneur en monomère libre inférieure ou égale à 50 ppm, procédé comportant au moins les séquences :
a) de préparation de la phase solvant,
b) de préparation de la phase aqueuse d'acide acrylique monomère(charge I)
c) de mise en suspension inverse du monomère et polymérisation I,
d) de préparation de la phase aqueuse d'acide acrylique monomère (charge Il),
e) d'absorption de la charge Il et de polymérisation Il,
f) de séparation du polymère,
caractérisé en ce que la séquence de séparation du polymère comprend au moins une opération de déshydratation azéotropique et une opération de séchage et qu'on introduit de l'eau oxygénée sur ou dans le polymère entre lesdites opérations de déshydratation et de séchage, à raison de 0,08 à 0,19 % d'eau oxygénée par rapport au polymère sec.

2. Procédé pour la réalisation de poudres superabsorbantes selon la revendication 1, caractérisé en ce que la charge I d'acide acrylique monomère est neutralisée à un taux supérieur à celui du polymère polyacrylique final, tandis que la charge Il d'acide acrylique monomère est neutralisée à un taux inférieur.

3. Procédé selon la revendication 2, caractérisé en ce que les charges d'acide acrylique monomère dans les phases b et d sont préparées en quantités égales, que le taux de neutralisation de la charge I est compris entre 90 et 100 % et que celui de la charge Il est compris entre 60 et 50 %.

4. Procédé selon l'une ou l'autre des revendications 1 à 3, caractérisé en ce que l'acide acrylique monomère de la première charge est mis en suspension inverse à l'aide de tensioactifs de type copolymères-blocs qui résultent de la réaction d'époxyalcanes sur les polyalkylèneglycols ou leurs monoesters

5. Procédé selon la revendication 4, caractérisé en ce que le tensioactif est un copolymère bloc polyéthylèneglycol/ dodécylglycol.

6. Procédé selon les revendications 4 ou 5, caractérisé en ce que l'absorption de la seconde charge de monomère est réalisée à une température voisine de 35°C.

7. Procédé selon l'une ou l'autre des revendications 1 à 3, caractérisé en ce que l'acide acrylique monomère de la charge I est mis en suspension inverse à l'aide d'un tensioactif polymérisable répondant à la formule générale:
R₁-O-(CH₂-CH₂-O)ₙ-R₂
- où R₁ est une chaîne hydrocarbonée à au moins 9 atomes de carbone,
- où R₂ est un groupement polymérisable, reste acryloyle, méthacryloyle ou maléoyle,
- et où le degré de condensation n en oxyde d'éthylène est compris entre 30 et 70.

8. Procédé selon la revendication 7, caractérisé en ce que le tensioactif est l'ester maléique de nonylphénol oxyéthyléné à 50 molécules d'oxyde d'éthylène, et qu'il est mis en solution dans la phase aqueuse.

9. Procédé selon les revendications 7 ou 8, caractérisé en ce que l'absorption de la seconde charge de monomère est réalisée à une température de 35 à 45°C.

10. Poudre d'acide polyacrylique neutralisé à un taux compris entre 65 et 95 %, de diamètre moyen compris entre 100 et 500 µm et de passant à 100 µm inférieur à 0,5 %, caractérisée en ce qu'elle est constituée de particules sphéroïdales à surface irrégulière et que sa teneur en monomères résiduels est tout au plus égale à 50 ppm.

## Patentansprüche

1. Verfahren zur Gewinnung von Wasser und wäßrige Flüssigkeiten superabsorbierenden Pulvern aus teilneutralisierter Polyacrylsäure mit einem Gehalt von 50 ppm oder weniger an freiem Monomerem, das mindestens die Schrittfolgen umfaßt:
a) Herstellung der Lösungsphase,
b) Herstellung der wäßrigen Acrylsäure-Monomer-Phase (Ansatz I),
c) Inverses Suspendieren des Monomeren und Polymerisation I,
d) Herstellung der wäßrigen Acrylsäure-Monomer-Phase (Ansatz II),
e) Absorption des Ansatzes II und Polymerisation II,
f) Abtrennung des Polymeren,
dadurch gekennzeichnet, daß die Abtrennung des Polymeren mindestens einen Schritt der azeotropen Dehydratisierung und einen Trocknungsschritt umfaßt und daß man zwischen den Schritten der Abtrennung und Dehydratisierung Wasserstoffperoxid in einer Menge von 0,08 bis 0,19 %, bezogen auf das trockene Polymere, auf das Polymere auf- oder in das Polymere einbringt.

2. Verfahren nach Anspruch 1, dadurch gekennzeichnet, daß der Ansatz I des Acrylsäure-Monomeren mit einem Prozentsatz über dem des endgültigen Acrylsäure-Polymeren neutralisiert wird, während der Ansatz II des Acrylsäure-Monomeren mit einem Prozentsatz unter dem des endgültigen Acrylsäurepolymeren neutralisiert wird.

3. Verfahren nach Anspruch 2, dadurch gekennzeichnet, daß die Ansätze von Acrylsäure-Monomerem in den Phasen b und d in gleichen Mengen zubereitet werden und daß der Prozentsatz der Neutralisation des Ansatzes I zwischen 90 und 100 und der des Ansatzes II zwischen 50 und 60 % liegt.

4. Verfahren nach einem der Ansprüche 1 bis 3, dadurch gekennzeichnet, daß das Acrylsäure-Monomere des ersten Ansatzes mit Hilfe von Tensiden vom Typ der Blockcopolymeren aus der Umsetzung von Epoxyalkanen mit Polyalkylenglykolen oder deren Monoestern in inverse Suspension gebracht wird.

5. Verfahren nach Anspruch 4, dadurch gekennzeichnet, daß das Tensid ein Blockcopolymeres von Polyalkylenglykol/Dodecylglykol ist.

6. Verfahren nach den Ansprüchen 4 oder 5, dadurch gekennzeichnet, daß die Absorption des zweiten Ansatzes bei einer Temperatur im Bereich von 35 °C vorgenommen wird.

7. Verfahren nach einem der Ansprüche 1 bis 3, dadurch gekennzeichnet, daß das Acrylsäure-Monomere des ersten Ansatzes mit Hilfe eines polymerisierbaren Tensids in inverse Suspension gebracht wird, das der allgemeinen Formel
R₁-O-(CH₂-CH₂-O)ₙ-R₂
entspricht
- wobei R₁ eine Kohlenwasserstoffkette mit wenigstens 9 Kohlenstoffatomen ist,
- wobei R₂ eine polymerisierbare Gruppierung mit einem Acryloyl-, Methacryloyl- oder Maleoylrest ist,
- und wobei der Kondensationsgrad des Ethylenoxids zwischen 30 und 70 liegt.

8. Verfahren nach Anspruch 7, dadurch gekennzeichnet, daß das Tensid der Maleinsäureester des mit 50 Mol Ethylenoxid oxethylierten Nonylphenols ist und daß es in wäßrige Lösung gebracht worden ist.

9. Verfahren nach den Ansprüchen 7 oder 8, dadurch gekennzeichnet, daß die Absorption des zweiten Ansatzes bei einer Temperatur von 35 bis 45 °C stattgefunden hat.

10. Pulver aus zu 65 bis 95 % neutralisierter Polyacrylsäure mit einem mittleren Durchmesser von 100 bis 500 um mit einem Siebanteil unter 100 µm von weniger als 0,5 %, dadurch gekennzeichnet, daß es aus kugeligen Teilchen mit unregelmäßiger Oberfläche besteht und daß sein Gehalt an Restmonomeren höchstens 50 ppm beträgt.

## Claims

1. Process for the production of powders which are superabsorbent for water and aqueous liquids and consist of partially neutralised polyacrylic acid having a free monomer content lower than or equal to 50 ppm, which process comprises at least the following sequences:
a) preparation of the solvent phase,
b) preparation of the aqueous phase of acrylic acid monomer (charge I),
c) bringing of the monomer into inverse suspension, and polymerisation I,
d) preparation of the aqueous phase of acrylic acid monomer (charge II),
e) absorption of charge II and polymerisation II and
f) isolation of the polymer,
characterised in that the sequence of isolation of the polymer comprises at least one azeotropic dehydration operation and one drying operation and that hydrogen peroxide is introduced onto or into the polymer between the said dehydration and drying operations, at a rate of 0.08 to 0.19 % of hydrogen peroxide with respect to the dry polymer.

2. Process for the production of superabsorbent powders according to Claim 1, characterised in that charge I of monomeric acrylic acid is neutralised to a degree greater than that of the final polyacrylic polymer, whilst charge II of monomeric acrylic acid is neutralised to a lower degree.

3. Process according to Claim 2, characterised in that the charges of monomeric acrylic acid in phases b and d are prepared in equal amounts, in that the degree of neutralisation of charge I is between 90 and 100 % and in that the degree of neutralisation of charge II is between 60 and 50 %.

4. Process according to one or other of Claims 1 to 3, characterised in that the monomeric acrylic acid of the first charge is brought into inverse suspension with the aid of surfactants of the block copolymer type, which result from the reaction of epoxyalkanes with polyalkylene glycols or with their monoesters.

5. Process according to Claim 4, characterised in that the surfactant is a polyethylene glycol/dodecyl glycol block copolymer.

6. Process according to Claim 4 or 5, characterised in that the absorption of the second monomer charge is carried out at a temperature of about 35°C.

7. Process according to one or other of Claims 1 to 3, characterised in that the monomeric acrylic acid of charge I is brought into inverse suspension with the aid of a polymerisable surfactant corresponding to the general formula:
R₁-O-(CH₂-CH₂-O)ₙ-R₂
where R₁ is a hydrocarbon chain having at least 9 carbon atoms,
where R₂ is a polymerisable group, namely an acryloyl, methacryloyl or maleoyl radical and
where the degree of condensation n in respect of ethylene oxide is between 30 and 70.

8. Process according to Claim 7, characterised in that the surfactant is the maleic acid ester of nonylphenol oxyethylenated with 50 molecules of ethylene oxide, and in that it is dissolved in the aqueous phase.

9. Process according to Claim 7 or 8, characterised in that the absorption of the second monomer charge is carried out at a temperature of 35 to 45°C.

10. Polyacrylic acid powder neutralised to a degree of between 65 and 95 %, having a mean diameter of between 100 and 500 µm, and with less than 0.5 % passing through a 100 µm screen, characterised in that it consists of spheroidal particles having an irregular surface and in that its residual monomer content is at most 50 ppm.
